(19) Europäisches Patentamt
European Patent Office
Office européen des brevets

(11) Publication number: 0 112 135 B1

(12) **EUROPEAN PATENT SPECIFICATION**

(45) Date of publication of patent specification: **13.01.88**

(51) Int. Cl.$^4$: **C 07 D 501/00**

(21) Application number: **83307505.4**

(22) Date of filing: **09.12.83**

# (54) Bromination of cephalosporins.

(30) Priority: **17.12.82 US 450707**

(43) Date of publication of application: **27.06.84 Bulletin 84/26**

(45) Publication of the grant of the patent: **13.01.88 Bulletin 88/02**

(84) Designated Contracting States: **BE CH DE FR IT LI LU NL SE**

(56) References cited:
**US-A-4 018 776**
**US-A-4 091 026**

(73) Proprietor: **ELI LILLY AND COMPANY**
**307, East McCarty Street**
**Indianapolis Indiana 46285 (US)**

(72) Inventor: **Cooper, Robin David Grey**
**6740 Dover Road**
**Indianapolis, IN 46220 (US)**

(74) Representative: **Crowther, Terence Roger et al**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

3-Halo-3-methylcephams are useful in the synthesis of 3-methyl-3-cephem antibiotics. Because of this, several proceses have been developed for the preparation of these compounds. Micetich and Morin, for instance, in *Tetrahedron Letters*, No. 13, pp. 979—982, 1976, disclosed the reaction of a thiazoline azetidinone with iodine and water to give the corresponding 3-iodo-3-methylcepham. This reference reports that the reaction was not general in that bromine and chlorine failed to behave as did iodine.

Foglio et al., in U.S. Patent No. 4,018,776, report an iodination process comprising reaction of a thiazoline azetidinone with iodine in the presence of a heavy metal oxide or a free radical initiator such as a peroxide. Nadelman et al., in U.S. Patent No. 3,932,398, disclose the reaction of a penicillin sulfoxide with a neutral or basic catalyst in a polyhaloalkane solvent to give a 3-halo-3-methylcepham. More recently, Yoshioka et al., in U.S. Patent No. 4,183,855, disclosed the synthesis of certain 3-halo-3-methyl-1-oxacephams from oxazoline azetidinone derivatives.

In this regard, this invention provides a new process for preparing 3-bromo-3-methylcephams.

In particular, this invention concerns a process for converting thiazoline azetidinones to 3-bromocephams. In accordance with the invention, there is provided a process for preparing a 3-bromocepham compound of Formula (I):

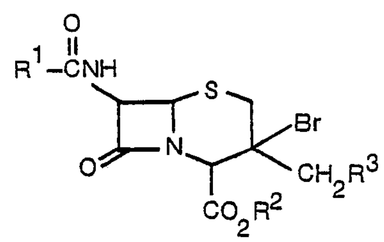

(I)

in which $R^1$ is a residue of an acyl group commonly employed in cephalosporins, $R^2$ is a carboxy protecting group, and $R^3$ is hydrogen, bromo, chloro, acetoxy, alkylthio, arylthio, tetrazolylthio, thiadiazolylthio

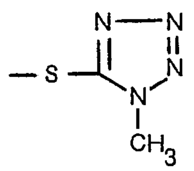

or

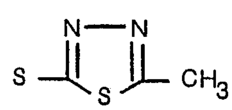

which comprises reacting a thiazoline acetidinone of Formula (II):

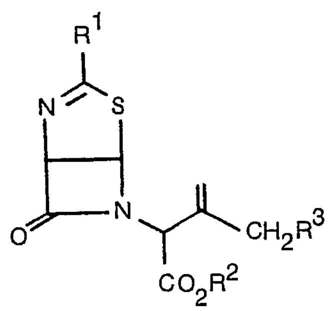

(II)

with a bromine source and dimethyl sulfoxide.

The process is preferably carried out employing a thiazoline azetidinone in which $R^1$ is phenoxymethyl, phenylmethyl, 2-thienylmethyl, chloromethyl, α-formyloxyphenylmethyl, or α-acetamidophenylmethyl; $R^2$ is p-nitrobenzyl, 2,2,2-trichloroethyl, methyl or diphenylmethyl; and $R^3$ is hydrogen or chloro.

In the above formulas, $R^1$ is defined as "an organic radical". This term refers to a monovalent group of an acyl residue of a carboxylic acid, less the acyl carbonyl moiety. $R^1$ is the monovalent organic radical resulting from the diminution of the carbonyl function of an acyl group derived from a carboxylic acid. These $R^1$ groups are well known in the cephalosporin art, and no novelty is asserted herein regarding the groups defined by $R^1$. Typical $R^1$ moieties may include

(a) $C_1$—$C_7$ alkyl, cyanomethyl, $C_1$—$C_6$ haloalkyl, 4-protected amino-4-protected carboxybutyl; or

(b) $C_1$—$C_6$ alkoxy, phenoxy, benzyloxy or 4-methoxybenzyloxy; or

(c) the group —$R^4$ in which $R^4$ is phenyl or substituted phenyl in which the substituents are 1 or 2 halogens, protected hydroxy, cyano, trifluoromethyl, $C_1$—$C_4$ alkyl, $C_1$—$C_4$ alkoxy, protected carboxy, protected carboxymethyl, protected hydroxymethyl, protected aminomethyl, or a bicyclic aryl group such as naphthyl, benzothienyl, substituted naphthyl or substituted benzothienyl; or

(d) a group of the formula $R^4$—$(O)_m$—$CH_2$— in which $R^4$ is as defined above, and m is 0 or 1; or

(e) a substituted arylalkyl group of the formula

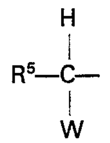 or 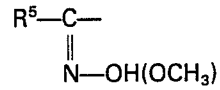

in which $R^5$ is $R^4$ as defined above, 2-thienyl, 3-thienyl, 2-protected aminothiazol-4-yl, 2-furyl or 3-furyl; W is protected hydroxy, protected carboxy, protected amino, or

(f) a heteroarylmethyl group of the formula $R^6—CH_2—$ in which $R^6$ is 2-thienyl, 3-thienyl, 2-protected aminothiazol-4-yl, 2-furyl, 3-furyl, 2-thiazolyl, 5-tetrazolyl, 1-tetrazolyl.

$R^2$ in the above formula is a carboxy protecting group.

$R^3$ in the above formula is hydrogen, chloro, acetoxy, alkylthio such as methylthio or *tert.*-butylthio, or arylthio such as phenylthio or tetrazolylthio, or thiadiazolylthio.

In the foregoing definitions, the term "$C_1—C_7$ alkyl" refers to methyl, ethyl, n-propyl, n-butyl, isobutyl, pentyl, n-hexyl, cyclohexyl, n-heptyl and other straight and branched aliphatic hydrocarbon chains.

The term "$C_1—C_6$ haloalkyl" refers to groups such as chloromethyl, bromomethyl, iodomethyl, 2-bromoethyl, 2-chloroethyl, 2-bromopropyl, 2-iodopropyl, 2-chlorobutyl, 2-bromo-2-methylpropyl, 2-bromobutyl, and 2-bromo-2-methylbutyl.

In this specification, the protecting group designation is omitted for simplicity in nomenclature, but it is understood that, in the description of the process of this invention, each carboxy, hydroxy or amino group is to be appropriately protected.

"Protected amino" refers to an amino group substituted with a commonly employed amino blocking group such as the tert-butoxycarbonyl group (t-BOC), the benzyloxycarbonyl group, the 4-methoxybenzyloxycarbonyl group, the 2,2,2-trichloroethoxycarbonyl group, or the dimethyl-*tert.*-butylsilyl group. The nature of such amino protecting groups is not critical as long as the protected amino functionality is stable under the reaction conditions described.

"Protected hydroxy" refers to any group stable under the reaction conditions for the synthesis of the 3-bromo-3-methylcepham compounds, but which is readily cleavable afterwards. Such groups may include the formyloxy group, the chloroacetoxy group, the benzhydryloxy group, the β-trimethylsilylethyloxy group, or the dimethyl-*tert.*-butylsilyl group.

The term "carboxy protecting group" or "protected carboxy" refers to a carboxy group which has been esterified with a commonly used carboxylic acid protecting ester group and is used to block or protect the carboxylic acid functionality while reactions involving other functional sites of the molecule are performed. Such protected carboxy groups are noted for their ease of cleavage to the corresponding carboxylic acid. Examples of carboxylic acid protecting groups may include tert-butyl, methyl, cyanomethyl, p-methoxybenzyl, diphenylmethyl, 2,4,6-trimethylbenzyl, or 2,2,2-trichloroethyl. The nature of such ester forming groups is not critical as long as the ester formed is stable under the reaction conditions described. Preferred carboxylic acid protecting groups are diphenylmethyl, 4-methoxybenzyl, and *p*-nitrobenzyl.

In these definitions, hydroxy, amino, and carboxy protecting groups are not exhaustively defined. The function of such groups is to protect the reactive functional groups during the preparation of the desired products and the groups then are to be removed without disrupting the remainder of the molecule. Such protecting groups are well known in the art and the use of other groups equally applicable to the process and compounds of the present invention, such as those described in J. F. W. McOmie, "Protective Groups in Organic Chemistry", Plenum Press, 1973, will be recognized as suitable.

When $R^4$ represents a substituted phenyl group, $R^4$ can be a mono or disubstituted halophenyl group such as 4-chlorophenyl, 2,6-dichlorophenyl, 2,5-dichlorophenyl, 3,4-dichlorophenyl, 3-chlorophenyl, 3-bromophenyl, 4-bromophenyl, 3,4-dibromophenyl, 3-chloro-4-fluorophenyl, or 2-fluorophenyl; a mono or dihydroxyphenyl group such as 4-hydroxyphenyl, 3-hydroxyphenyl, or 2,4-dihydroxyphenyl (where all hydroxy groups are protected); a cyanophenyl group, for example, 4-cyanophenyl; a mono or disubstituted lower alkylphenyl group such as 4-methylphenyl, 2,4-dimethylphenyl, 2-methylphenyl, 4-isopropylphenyl, 4-ethylphenyl, or 3-n-propylphenyl; a mono or disubstituted lower alkylphenyl ether such as 2,6-dimethoxyphenyl, 4-methoxyphenyl, 3-ethoxyphenyl, 4-isopropoxyphenyl, 4-tert-butoxyphenyl, or 3-ethoxy-4-methoxyphenyl. Also, $R^4$ may represent disubstituted phenyl groups in which the substituents can be different, for example, 3-methyl-4-hydroxyphenyl, 3-chloro-4-hydroxyphenyl, 2-methoxy-4-bromophenyl, 4-ethyl-2-hydroxyphenyl, 3-hydroxy-4-nitrophenyl, or 2-hydroxy-4-chlorophenyl.

The process of this invention converts a thiazoline azetidinone to a cephalosporin derivative having an organic acyl group defined as

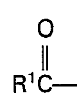

attached to the 7-amino group.

Illustrative of the acyl groups,

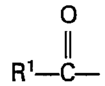

in which $R^1$ is $C_1$—$C_7$ alkyl, or $C_1$—$C_6$ haloalkyl, are acetyl, propionyl, butyryl, hexanoyl, chloroacetyl, or bromoacetyl.

Representative of the acyl groups

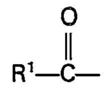

in which $R^1$ is phenyl or substituted phenyl are benzoyl, 2,6-dimethoxybenzoyl, 4-chlorobenzoyl, 4-methylbenzoyl, 3,4-dichlorobenzoyl, 4-cyanobenzoyl, 3-bromobenzoyl, or 3-protected aminobenzoyl.

Illustrative of the acyl groups

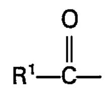

in which $R^1$ is a group of the formula $R^4$—$(O)_m$—$CH_2$—, m is 0 and $R^4$ is phenyl or substituted phenyl, are phenylacetyl, 4-chlorophenylacetyl, 3-protected hydroxyphenylacetyl, 3-cyanophenylacetyl, 4-acetoxy-3-methylphenylacetyl, 4-bromophenylacetyl, 4-ethoxyphenylacetyl, or 3,4-dimethoxyphenylacetyl; and when m is 1, representative groups may be phenoxyacetyl, 3-hydroxyphenoxyacetyl, 4-chloropheoxyacetyl, 3,4-dichlorophenoxyacetyl, 2-chlorophenoxyacetyl, 4-methoxyphenoxyacetyl, 2-ethoxyphenylacetyl, 3,4-dimethylphenoxyacetyl, 4-isopropylphenoxyacetyl, or 3-cyanophenoxyacetyl.

Illustrative of the acyl groups in which $R^1$ is a substituted arylalkyl group of the formula

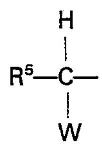

are the carboxy substituted acyl groups such as the 2-carboxy-2-phenylacetyl group of the formula

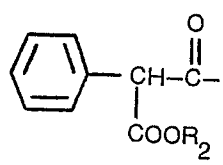

and similar groups in which the phenyl ring is substituted, such as 2-carboxy-2-(4-chlorophenyl)acetyl, 2-carboxy-2-(4-methoxyphenyl)acetyl, 2-carboxy-2-(2-thienyl)acetyl, 2-carboxy-2-(4-methylphenyl)acetyl, 2-carboxy-2-(4-(carboxymethyl)phenyl)acetyl, or 2-carboxy-2-(4-(formyloxymethyl)phenyl)acetyl.

Representative of the acyl groups in which $R^1$ is a hydroxy substituted arylalkyl group are 2-hydroxy-2-(4-methoxyphenyl)acetyl, 2-hydroxy-2-(3-chloro-4-hydroxyphenyl)acetyl, 2-hydroxy-2-(3-bromophenyl)-acetyl, 2-hydroxy-2-(3,5-dichloro-4-hydroxyphenyl)acetyl, 2-hydroxy-2-(3-chloro-4-methoxyphenyl)acetyl, 2-hydroxy-2-(3-chlorophenyl)acetyl, 2-hydroxy-2-(4-aminomethylphenyl)acetyl, or 2-hydroxy-2-(3-thienyl)-acetyl; of course all hydroxy groups bear a hydroxy protecting group during the process of this invention.

Representative groups in which $R^1$ is an amino substituted arylalkyl group may include 2-amino-2-phenylacetyl, 2-amino-2-(4-cyanophenyl)acetyl, or 2-amino-2-(4-hydrophenyl)acetyl.

Representative of the acyl group

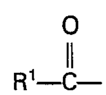

in which $R^1$ is a heteroarylmethyl group of the formula $R^6—CH_2—$ are 2-thienylacetyl, 3-thienylacetyl, 2-furylacetyl, 2-thiazolylacetyl, 1-tetrazolylacetyl, or 5-tetrazolylacetyl.

In the above formulas, $R^3$ is hydrogen, bromo, chloro, acetoxy, alkylthio or arylthio. "Arylthio" includes groups such as phenylthio as well as a heterocyclic ring system selected from

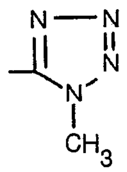

or

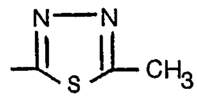

attached through a sulfur atom.

In accordance with the invention, a thiazoline azetidinone is reacted with a bromine source in dimethyl sulfoxide. The term "bromine source" means any compound containing bromine that is capable of being oxidized by dimethyl sulfoxide to provide $Br^+$ or its equivalent. Many bromine containing compounds are known to be subject to oxidation by dimethyl sulfoxide. Nace and Monagle, for instance, described the oxidation of primary alkyl halides by dimethyl sulfoxide to give aldehydes, and halide$^+$ as a by-product: *J. Org. Chem., 24,* 1972 (1959). Any such primary alkyl bromides can be employed in the present process; all that is required is that $Br^+$ cations be generated so as to react with the thiazoline azetidinone. Typical compounds employed as bromine sources for this invention include liquid bromine (i.e. $Br_2$); hydrogen bromide; alkenyl bromides such as allyl bromide; arylalkyl bromides such as phenylmethyl bromide, 2-phenylethyl bromide, or 2-naphthylethyl bromide; and aroylalkyl bromides such as benzoylmethyl bromide, 4-methylbenzoylmethyl bromide, or 2-(4-bromobenzoyl)ethyl bromide. Preferred bromine sources include liquid bromine ($Br_2$), benzoylmethyl bromide and hydrobromic acid.

The process of the invention generally employs dimethyl sulfoxide in excessive amounts so as to function as reactant and as solvent. Other solvents can be employed if desired, however, for instance in conjunction with dimethyl sulfoxide. Other solvents which may be employed include water, toluene, N,N-dimethylformamide, dichloromethane, benzene, or xylene. When such co-solvents are employed, the dimethyl sulfoxide is present in about a one molar excess relative to the thiazoline azetidinone. The preferred solvent, however, is dimethyl sulfoxide used both as reactant and solvent. Another preferred solvent system is a mixture of dimethylsulfoxide and water.

The bromine source, such as liquid bromine or benzoylmethyl bromide, generally is employed in approximately equimolar amounts relative to the thiazoline azetidinone. If desired, however, amounts ranging from about 1 to 10 molar excess can be employed without adverse consequences.

The process generally is performed at a temperature of about 0 to 50°C. A preferred embodiment of the invention comprises carrying out the reaction at a temperature of about 20 to about 30°C., and ideally at about room temperature of approximately 24°C.

The process of the invention appears to be improved in some instances by the addition of a catalytic amount of acid to the reaction mixture. Typical acids which may be used include p-toluenesulfonic acid (generally as the monohydrate), acetic acid, or hydrobromic acid. The use of an added acid, however, is not a critical aspect of the present process.

The reaction generally is complete within about forty-eight hours, although longer reaction times do not appear detrimental and may be employed if desired. Typically, the reaction is carried out overnight, for about twelve to sixteen hours.

Upon completion of the process, the 3-bromo-3-methylcepham product can be isolated conveniently by routine methods. Normally, the reaction solvent is removed by evaporation under reduced pressure and the resulting product is simply purified by standard techniques such as chromatography or crystallization.

The 3-bromo-3-methylcephams prepared by the process of this invention are useful in the synthesis of cephalosporin antibiotics. For example, the 3-bromo-3-methylcephams are transformed easily to the corresponding $\Delta^3$-cephems by treatment with an inorganic or organic base such as potassium hydroxide, sodium carbonate, aliphatic or aromatic amines, quaternary alkylammonium bases, or basic ion exchange resins. This conversion is shown in the following scheme:

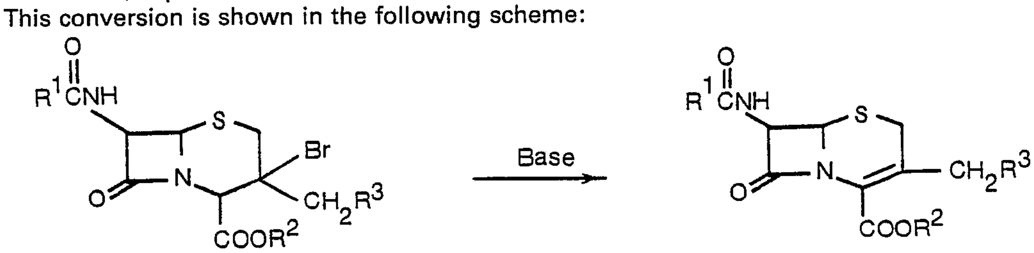


in which $R^1$, $R^2$ and $R^3$ are as defined previously. Removal of any protecting groups (for example $R^2$, the carboxy protecting group) by routine methods affords the corresponding 3-methyl-3-cephem-4-carboxylic acid or salt, the cephalosporin antibiotic. The latter compounds are well known in the art and are useful in the treatment of bacterial infections in humans and animals.

To more fully illustrate the invention, the following non-limiting examples are provided:

Example 1

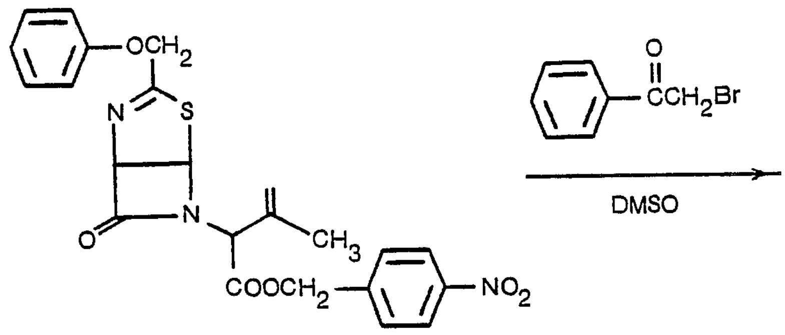



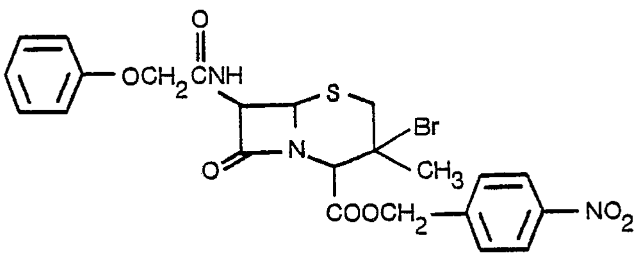

A solution of 466 mg. (1 mmole) of the thiazoline azetidinone in 5 ml. of dimethyl sulfoxide (DMSO) containing 200 mg. of benzoylmethyl bromide was stirred at 24°C. for sixteen hours. Thin layer chromatographic analysis indicated complete reaction. The solvent was removed from the reaction mixture by evaporation under reduced pressure to provide p-nitrobenzyl 7-phenoxyacetamido-3-bromo-3-methylcepham-4-carboxylate.

NMR ($CDCl_3$) δ 1.85 (3H, s, $CH_3$); δ 2.75, 3.55 (2H, $q_{AB}$, —$CH_2S$); δ 4.94 (1H, s); δ 5.36 (1H, d); δ 5.66, 5.79 (1H, 7-H).

Removal of the p-nitrobenzyl protecting group would produce the free acid, 7-phenoxyacetamido-3-bromo-3-methylcepham-4-carboxylic acid. IR ($CHCl_3$): 3400, 1780, 1739, 1690 $cm^{-1}$.

Example 2

The procedure of Example 1 was repeated, except that the reaction was conducted in 10 ml. of DMSO and 1 ml. of water. Following evaporation of the solvent mixture, the product was purified by preparative TLC to provide 304 mg. of p-nitrobenzyl 7-phenoxyacetamido-3-bromo-3-methylcepham-4-carboxylate.

Example 3

The procedure of Example 1 was repeated except that the reaction was carried out in the presence of 50 mg. of p-toluenesulfonic acid monohydrate. Normal isolation and purification by preparative TLC gave 270 mg. of p-nitrobenzyl 7-phenoxyacetamido-3-bromo-3-methylcepham-4-carboxylate.

Example 4

The procedure of Example 3 was repeated except that the reaction was carried out over a forty hour period. Preparative TLC using silica gel plates (3:1 ethyl acetate:benzene (v/v)) following evaporation of the solvent from the appropriate fractions, provided 381 mg. of p-nitrobenzyl 7-phenoxyacetamido-3-bromo-3-methylcepham-4-carboxylate.

Example 5

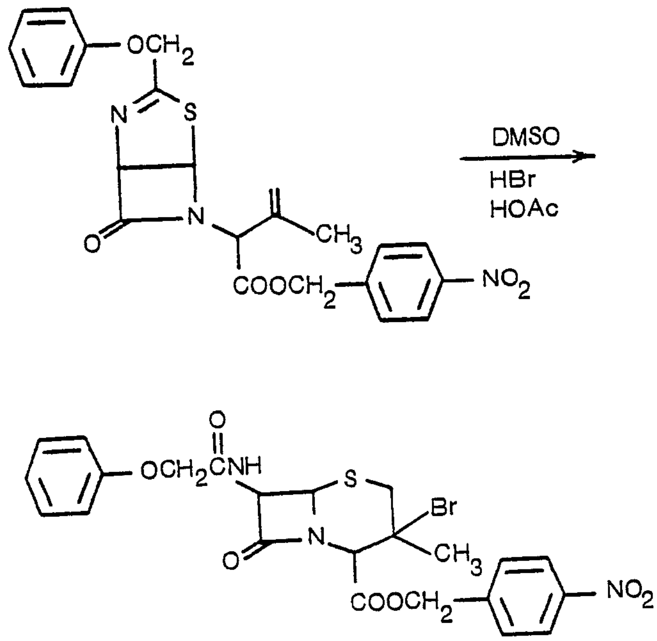


A solution of 466 mg. (1 mmole) of the thiazoline azetidinone in 5 ml. of DMSO containing 10 mg. of hydrobromic acid and 1.0 mg. of glacial acetic acid was stirred for sixteen hours at room temperature. Thin layer chromatographic analysis demonstrated that the only product was p-nitrobenzyl 7-phenoxyacetamido-3-bromo-3-methylcepham-4-carboxylate.

Example 6

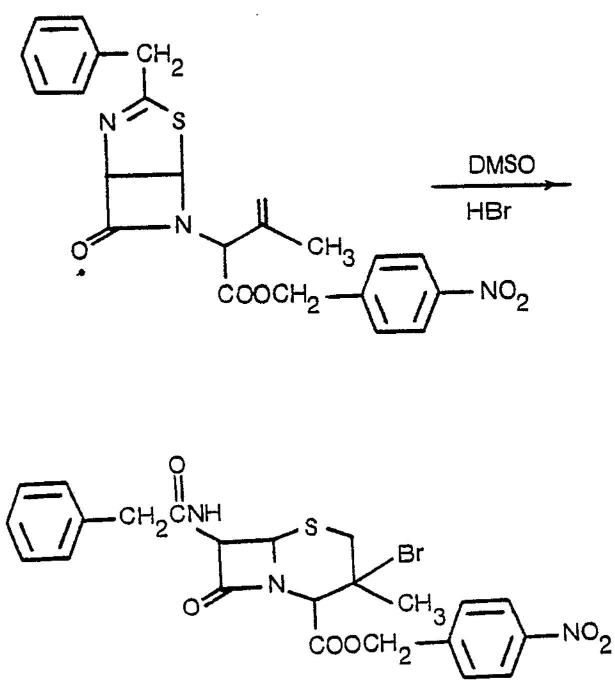


A solution of 253 mg. of the thiazoline azetidinone in 1 ml. of DMSO containing 50 mg. of 48% aqueous hydrobromic acid was stirred at 24°C. for sixteen hours. Evaporation of the reaction solvent under reduced pressure afforded 149 mg. of yellow foam identified as p-nitrobenzyl 7-phenylacetamido-3-bromo-3-methylcepham-4-carboxylate.

The melting point of 7-phenylacetamido-3-bromo-3-methylcepham-4-carboxylate is 136—137.5°C.

Example 7

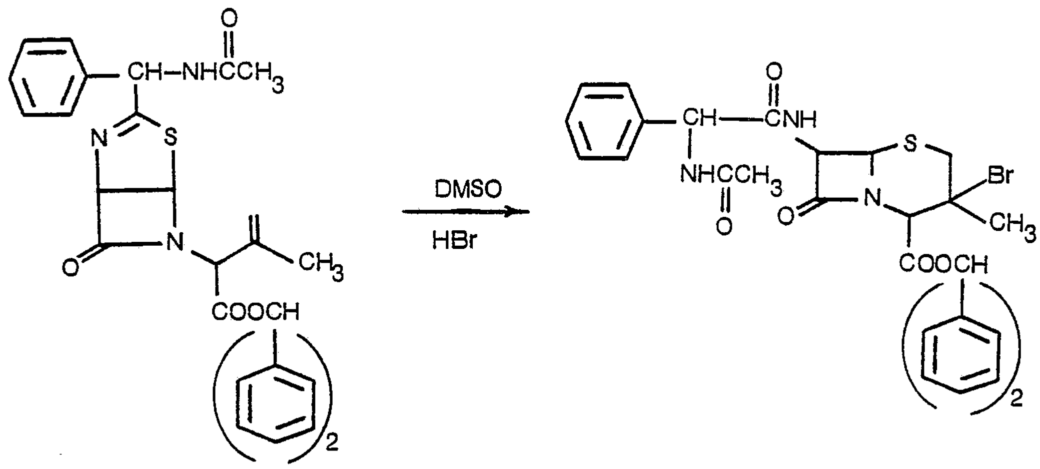


A solution of 230 mg. of the thiazoline azetidinone in 1 ml. of DMSO containing 34 mg. of 48% aqueous hydrobromic acid was stirred at room temperature for sixteen hours. Removal of the solvent by evaporation under reduced pressure provided 214 mg. that was identified as benzhydryl 7-(α-acetamido)-phenylacetamido-3-bromo-3-methylcepham-4-carboxylate. Further purification by preparative TLC gave 63 mg. of the 3-bromo-3-methylcepham.

NMR ($CDCl_3$) δ 2.0 (3H, s, $CH_3$); δ 2.13 (s, 3H); δ 3.35 (2H); δ 4.92 (1H, s); δ 5.10 (1H, d); δ 5.44 (1H, d); δ 5.75 (H, q); δ 6.87 (1H, s); δ 7.4 (15H).

Example 8

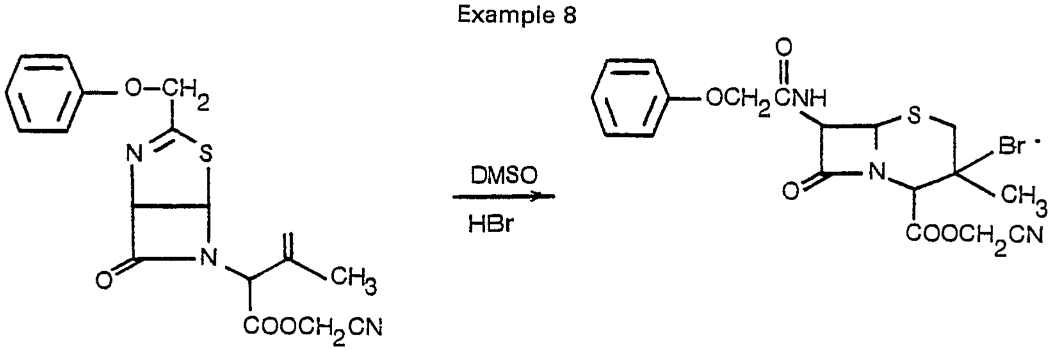


A solution of 120 mg. of the thiazoline azetidinone in 1 ml. of DMSO containing 0.02 ml. of 48% aqueous hydrobromic acid was stirred at 24°C. for sixteen hours to provide, following isolation by the method of Example 7, 68 mg. of cyanomethyl 7-phenoxyacetamido-3-bromo-3-methylcepham-4-carboxylate.

NMR ($CDCl_3$) δ 2.2 (3H, s, $CH_3$); δ 3.41 (2H, q); δ 4.39 (2H, s); δ 4.75 (1H, s); δ 4.83 (2H, q).

Example 9

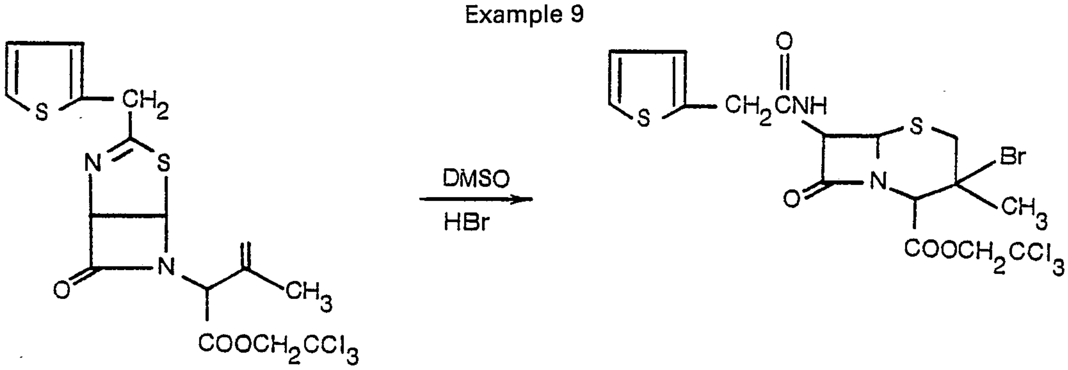


A solution of 190 mg. of the thiazoline azetidinone in 1 ml. of DMSO containing 0.03 ml. of 48% aqueous hydrobromic acid was stirred at room temperature for sixteen hours. THe solvent was removed by evaporation under reduced pressure to give 186 mg. of 2,2,2-trichloroethyl 7-(2-thiophene)acetamido-3-bromo-3-methylcepham-4-carboxylate.

NMR ($CDCl_3$) δ 1.98 (3H, s, $CH_3$); δ 3.15 (2H, quartet); δ 3.83 (2H, s); δ 4.82 (2H, s); δ 4.97 (1H, s); δ 5.29 (1H, doublet); δ 5.60 (1H, quartet); 6.8—7.3 (4H, multiplet, aromatic, NH).

Example 10

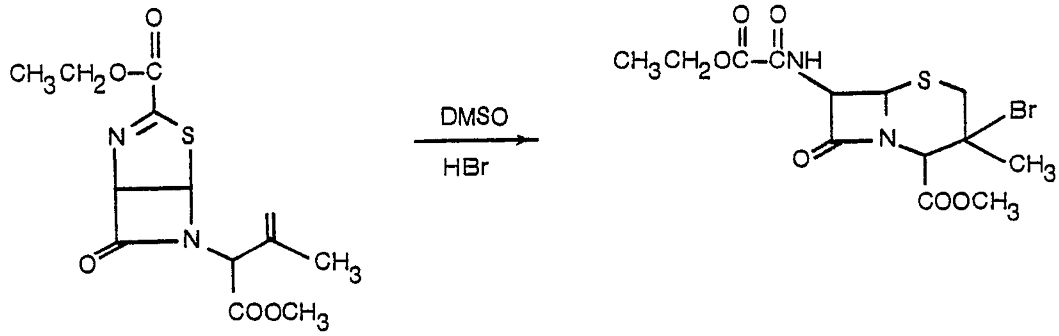


A solution of 312 mg. of the thiazoline azetidinone in 2 ml. of DMSO containing 0.106 ml. of 48% aqueous hydrobromic acid was stirred at room temperature for sixteen hours. Removal of the solvent by evaporation under reduced pressure gave 278 mg. of methyl 7-ethoxycarbonylformamido-3-bromo-3-methylcepham-4-carboxylate.

NMR ($CDCl_3$) δ 1.2 (3H, triplet J=7Hz); δ 1.90 (3H, s, $CH_3$); δ 3.22 (2H, quartet, J=15Hz); δ 3.81 (3H, s, $CH_3$ ester); 4.40 (2H, quartet J=7Hz); δ 4.85 (1H, s); δ 5.27 (1H, s, J=4Hz); δ 5.60 (1H, quartet); δ 8.15 (1H doublet).

**Claims**

1. A process for preparing a 3-bromo-3-methylcepham of Formula (I):

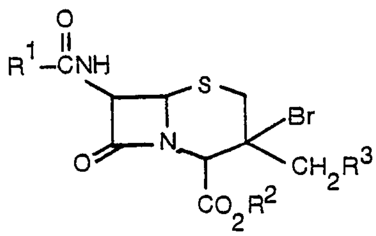

(I)

in which

$R^1$ is a residue of an acyl group commonly employed in cephalosporins;

$R^2$ is a carboxy protecting group;

$R^3$ is hydrogen, bromo, chloro, acetoxy, alkylthio, arylthio, tetrazolylthio, thiadiazolylthio,

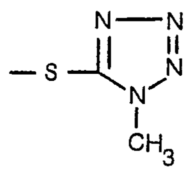

or

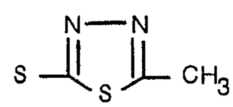

which comprises reacting a thiazoline azetidinone of Formula (II):

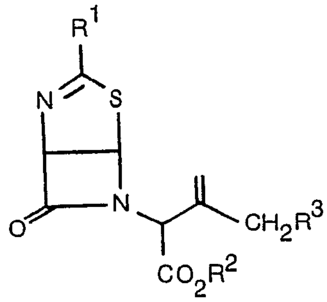

(II)

with a bromine source and dimethyl sulfoxide.

2. A process as claimed in Claim 1 in which $R^1$ is

(a) $C_1$—$C_7$ alkyl, cyanomethyl, $C_1$—$C_6$ haloalkyl, 4-protected amino-4-protected carboxybutyl; or

(b) $C_1$—$C_6$ alkoxy, phenoxy, benzyloxy or 4-methoxybenzyloxy; or

(c) the group —$R^4$ in which $R^4$ is phenyl or substituted phenyl in which the substituents are 1 or 2 halogens, protected hydroxy, cyano, trifluoromethyl, $C_1$—$C_4$ alkyl, $C_1$—$C_4$ alkoxy, protected carboxy,

protected carboxymethyl, protected hydroxymethyl, protected aminomethyl, or a bicyclic aryl group selected from naphthyl, benzothienyl, substituted naphthyl or substituted benzothienyl; or

(d) a group of the formula $R^4$—$(O)_m$—$CH_2$— in which $R^4$ is as defined above, and m is 0 or 1; or

(e) a substituted arylalkyl group of the formula

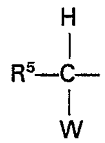 or 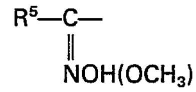

in which $R^5$ is $R^4$ as defined above, 2-thienyl, 3-thienyl, 2-protected aminothiazol-4-yl, 2-furyl or 3-furyl; W is protected hydroxy, protected carboxy, protected amino, or

(f) a heteroarylmethyl group of the formula $R^6$—$CH_2$— in which $R^6$ is 2-thienyl, 3-thienyl, 2-protected aminothiazol-4-yl, 2-furyl, 3-furyl, 2-thiazolyl, 5-tetrazolyl, 1-tetrazolyl.

3. A process as claimed in Claim 1 or 2 in which $R^2$ is selected from *tert.*-butyl, 2,2,2-trichloroethyl, cyanomethyl, methyl, p-methoxybenzyl, diphenylmethyl, 2,4,6-trimethylbenzyl, or p-nitrobenzyl.

4. A process as claimed in any one of Claims 1 to 3 wherein $R^3$ is hydrogen, chloro, acetoxy,

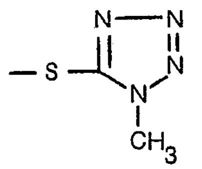 or 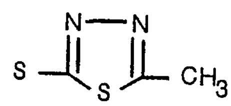

5. A process as claimed in any of Claims 1 to 4 wherein $R^1$ is phenoxymethyl, 2-thiophenemethyl, ethoxycarbonyl, chloromethyl α-acetamidophenylmethyl.

6. A process as claimed in Claim 4 in which $R^3$ is hydrogen or chloro.

7. A process as claimed in any one of Claims 1 to 6 in which the bromine source is hydrobromic acid.

8. A process as claimed in any one of Claims 1 to 6 in which the bromine source is benzoylmethyl bromide.

9. A process as claimed in any one of Claims 1 to 6 in which the bromine source is $Br_2$.

**Patentansprüche**

1. Verfahren zur Herstellung von 3-Brom-3-methylcephamen der Formel (I)

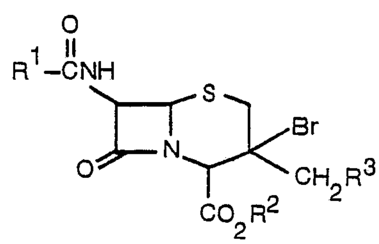

(I)

worin

$R^1$ der Rest einer bei Cephalosporin üblichen Acylgruppe ist,

$R^2$ eine Carboxyschutzgruppe bedeutet und

$R^3$ Wasserstoff, Brom, Chlor, Acetoxy, Alkylthio, Arylthio, Tetrazolylthio, Thiadiazolylthio

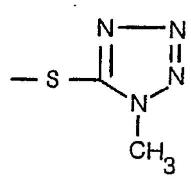 oder 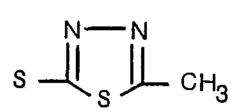

bedeutet,

dadurch gekennzeichnet, daß man ein Thiazolinazetidinon der Formel (II)

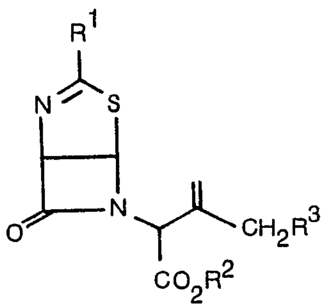

(II)

mit einer Bromquelle und mit Dimethylsulfoxid umsetzt.

2. Verfahren nach Anspruch 1, dadurch gekennzeichnet, daß $R^1$ folgende Bedeutungen hat

(a) $C_1$—$C_7$-Alkyl, Cyanomethyl, $C_1$—$C_6$-Halogenalkyl oder 4-Amino-4-carboxybutyl mit jeweils geschützter Amino- und Carboxygruppe oder

(b) $C_1$—$C_6$-Alkoxy, Phenoxy, Benzyloxy oder 4-Methoxybenzyloxy oder

(c) die Gruppe —$R^4$, worin $R^4$ Phenyl oder substituiertes Phenyl ist, wobei die Substituenten ein oder zwei Halogenatome, geschütztes Hydroxy, Cyano, Trifluormethyl, $C_1$—$C_4$-Alkyl, $C_1$—$C_4$-Alkoxy, geschütztes Carboxy, geschütztes Carboxymethyl, geschütztes Hydroxymethyl, geschütztes Aminomethyl oder eine bicyclische Arylgruppe ausgewählt aus Naphthyl, Benzothienyl, substituiertem Naphthyl oder substituiertem Benzothienyl bedeuten, oder

(d) eine Gruppe der Formel

$$R^4—(O)_m—CH_2—$$

worin $R^4$ wie oben definiert ist und m für 0 oder 1 steht, oder

(e) eine substituierte Arylalkylgruppe der Formel

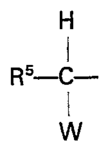 oder 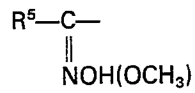

worin $R^5$ für den oben definierten Substituenten $R^4$, 2-Thienyl, 3-Thienyl, 2-Aminothiazol-4-yl mit geschützter Aminogruppe, 2-Furyl oder 3-Furyl bedeutet und W für geschütztes Hydroxy, geschütztes Carboxy oder geschütztes Amino steht, oder

(f) eine Heteroarylmethylgruppe der Formel

$$R^6—CH_2—$$

worin $R^6$ für 2-Thienyl, 3-Thienyl, 2-Aminothiazol-4-yl mit geschützter Aminogruppe, 2-Furyl, 3-Furyl, 2-Thiazolyl, 5-Tetrazolyl oder 1-Tetrazolyl steht.

3. Verfahren nach Anspruch 1 oder 2, dadurch gekennzeichnet, daß $R^2$ ausgewählt ist aus t-Butyl, 2,2,2-Trichlorethyl, Cyanomethyl, Methyl, p-Methoxybenzyl, Diphenylmethyl, 2,4,6-Trimethylbenzyl oder p-Nitrobenzyl.

4. Verfahren nach irgendeinem der Ansprüche 1 bis 3, dadurch gekennzeichnet, daß $R^3$ Wasserstoff, Chlor, Acetoxy,

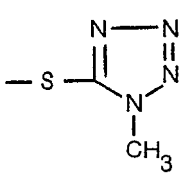 oder 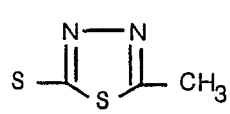

ist.

5. Verfahren nach irgendeinem der Ansprüche 1 bis 4, dadurch gekennzeichnet, daß $R^1$ Phenoxymethyl, 2-Thiophenmethyl, Ethoxycarbonyl, Chlormethyl oder α-Acetamidophenylmethyl ist.

6. Verfahren nach Anspruch 4, dadurch gekennzeichnet, daß $R^3$ Wasserstoff oder Chlor ist.

7. Verfahren nach irgendeinem der Ansprüche 1 bis 6, dadurch gekennzeichnet, daß die Bromquelle Bromwasserstoffsäure ist.

8. Verfahren nach irgendeinem der Ansprüche 1 bis 6, dadurch gekennzeichnet, daß die Bromquelle Benzoylmethylbromid ist.

9. Verfahren nach irgendeinem der Ansprüche 1 bis 6, dadurch gekennzeichnet, daß die Bromquelle $Br_2$ ist.

**Revendications**

1. Procédé de préparation d'un 3-bromo-3-méthylcépham de formule (I):

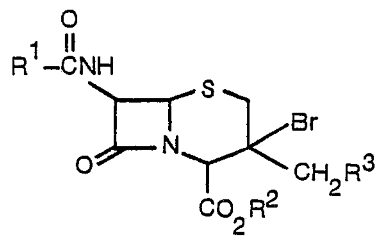

(I)

dans laquelle

$R^1$ représente un radical d'un groupe acyle habituellement utilisé dans des céphalosporines;

$R^2$ représente un groupe protecteur du groupe carboxyle;

$R^3$ représente un atome d'hydrogène, un atome de brome, un atome de chlore, un groupe acétoxy, un groupe alkylthio, un groupe arylthio, un groupe tétrazolylthio, un groupe thiadiazolylthio, un groupe

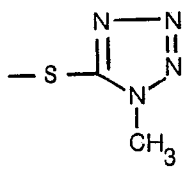 ou un groupe 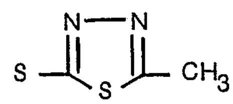

caractérisé en ce qu'il consiste à faire réagir une thiazoline-azétidinone de formule (II):

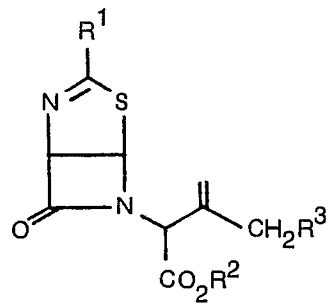

(II)

avec une source de brome et du diméthylsulfoxyde.

2. Procédé selon la revendication 1, dans lequel $R^1$ représente:

(a) un groupe alkyle en $C_1$—$C_7$, un groupe cyanométhyle, un groupe halo-alkyle en $C_1$—$C_6$, un groupe 4-amino protégé-4-carboxy protégé-butyle; ou

(b) un groupe alcoxy en $C_1$—$C_6$, un groupe phénoxy, un groupe benzyloxy ou un groupe 4-méthoxy-benzyloxy; ou

(c) le groupe —$R^4$ dans lequel $R^4$ représente un groupe phényle ou un groupe phényle substitué dans lequel les substituants sont 1 ou 2 atomes d'halogènes, un groupe hydroxyle protégé, un groupe cyano, un groupe trifluorométhyle, un groupe alkyle en $C_1$—$C_4$, un groupe alcoxy en $C_1$—$C_4$, un groupe carboxyle protégé, un groupe carboxy protégé-méthyle, un groupe hydroxy protégé-méthyle, un groupe amino protégé-méthyle ou un groupe aryle bicyclique choisi parmi un groupe naphtyle, un groupe benzothiényle, un groupe naphtyle substitué ou un groupe benzothiényle substitué; ou

(d) un groupe de formule $R^4$—$(O)_m$—$CH_2$— dans lequel $R^4$ a la signification définie ci-dessus, tandis que m représente 0 ou 1; ou

(e) un groupe arylalkyle substitué de formule:

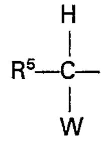 ou 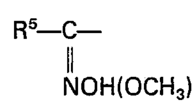

où $R^5$ représente $R^4$ tel que défini ci-dessus, un groupe 2-thiényle, un groupe 3-thiényle, un groupe 2-amino protégé-thiazol-4-yle, un groupe 2-furyle ou un groupe 3-furyle; W représente un groupe hydroxyle protégé, un groupe carboxyle protégé ou un groupe amino protégé, ou

(f) un groupe hétéro-arylméthyle de formule $R^6$—$CH_2$— où $R^6$ représente un groupe 2-thiényle, un

groupe 3-thiényle, un groupe 2-amino protégé-thiazol-4-yle, un groupe 2-furyle, un groupe 3-furyle, un groupe 2-thiazolyle, un groupe 5-tétrazolyle, ou un groupe 1-tétrazolyle.

3. Procédé selon la revendication 1 ou 2, dans lequel $R^2$ est choisi parmi le groupe *tert*-butyle, le groupe 2,2,2-trichloréthyle, le groupe cyanométhyle, le groupe méthyle, le groupe p-méthoxybenzyle, le groupe diphénylméthyle, le groupe 2,4,6-triméthylbenzyle ou le groupe p-nitrobenzyle.

4. Procédé selon l'une quelconque des revendications 1 à 3, dans lequel $R^3$ représente un atome d'hydrogène, un atome de chlore, un groupe acétoxy, un groupe

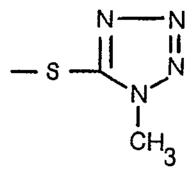 ou un groupe 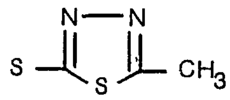

5. Procédé selon l'une quelconque des revendications 1 à 4, dans lequel $R^1$ représente le groupe phénoxyméthyle, le groupe 2-thiophène-méthyle, le groupe éthoxycarbonyle, le groupe chlorométhyle ou le groupe α-acétamidophénylméthyle.

6. Procédé selon la revendication 4, dans lequel $R^3$ représente un atome d'hydrogène ou un atome de chlore.

7. Procédé selon l'une quelconque des revendications 1 à 6, dans lequel la source de brome est l'acide bromhydrique.

8. Procédé selon l'une quelconque des revendications 1 à 6, dans lequel la source de brome est le bromure de benzoylméthyle.

9. Procédé selon l'une quelconque des revendications 1 à 6, dans lequel la source de brome est $Br_2$.